# EUROPEAN PATENT SPECIFICATION

(11) **EP 1 234 564 B1**
(45) Date of publication and mention of the grant of the patent: **19.04.2006**
(21) Application number: 01114559.6
(22) Date of filing: 18.06.2001
(51) Int. Cl.: A61F 13/551

(54) **Envelope for containing adhesive sanitary towels for women and production process**
Verpackung für selbstklebende Damenbinden und Verfahren zur dessen Herstellung
Enveloppe pour les Serviettes hygiéniques auto-adhésives et la méthode de fabrication correspondante

(30) Priority: 27.02.2001 IT BG010006
(43) Date of publication of application: 28.08.2002
(73) Proprietor: Plastik S.p.A., 24061 Albano S. Alessandro (Bergamo) (IT)
(72) Inventor: Cattaneo, Gianangelo, 24020 Scanzorosciate (Bergamo) (IT)
(74) Representative: Petraz, Gilberto Luigi

(56) References cited:
- GB-A- 2 273 279
- US-A- 5 972 473
- US-B1- 6 186 993

## Description

This invention relates to a envelope for containing adhesive sanitary towels for women, its constituent material and production process.

Modern sanitary towels 3 for women are known to consist generally of two thin films A, B of polyethylene, joined together at their edges to enclose absorbent material. Said sanitary towels have a shape suitable for adhering to the pantie interior in the "critical region" and have their sides defined by the two said films B and A, which are of different formation in order to be able respectively to adhere to the fabric of the pantie interior and constitute a barrier effect, and to allow the physiological humours to pass to the internal material provided for their retention. This adhesion of the sanitary towel structure to the pantie interior is achieved by a layer C of adhesive or sticky material, for example of the type D3347 of H.B. FULLER - SICAM. This sticky material has to remain covered, in order to prevent it adhering to foreign bodies, to reduce the evaporation of any solvents, and to avoid other chemical or physical phenomena which could otherwise prejudice its adhesive properties at the time of use. This covering for the sticky material layer C must be of substances which adhere to it with only minimum force, making it easy to remove the covering. This minimum adhesion force must however be sufficient to ensure that the covering remains securely positioned, so that it can perform its function. The material best used for this removable covering is silicone, generally applied on a strip of paper (silicone-coated paper) as a rigid support. With this arrangement, the sanitary towel structure is housed freely within a containing envelope 4. Said envelope is provided as a packaging element for individual sanitary towel structures for once-only use. After its contents have been removed, said envelope can be preserved to contain the used sanitary towel, while awaiting a suitable place for its disposal. The aforegoing description highlights the required characteristic of said envelope, namely an extremely flexible and silent, extremely thin, impermeable and opaque material of construction. These properties supplement the requirement to create containing envelopes which are increasingly more perfect, increasingly more functional and increasingly more economical.

A version of the silicone-coated paper strip to be removed before use is one in which one side of said strip is secured to the interior of the envelope by a second adhesive more sticky than that provided on the other side to protect the adhesive material which has to adhere to the pantie interior. This solution has the advantage of automatically detaching the silicone-coated paper by the same operation which extracts the sanitary towel from its envelope; however it has the drawback of a envelope made "bulky" by the presence of the silicone-coated paper adhering to it. The state of the art also comprises a polyethylene envelope version the interior of which has a surface treated by a silicone spreading process (described in US-A-5181610 and US-A-5474818). A method for producing polyethylene film for constructing said containing envelopes for usual sanitary towels is also known, achieved by coextrusion of silicone-coated film.

Those envelopes produced from silicone-coated polyethylene film by the aforelisted methods demonstrate a non-stick capacity the maximum value of which can be defined as excessive. In this respect, the glue for adhering to the pantie interior may be excessive in quantity or not perfectly applied to the sanitary towel, such as to adhere to its fabric part so strongly as to leave residues attached to it. This is because the usual production methods do not enable the quantity of adhesive applied to the sanitary towel to be controlled with the necessary precision, with the result that it may be transferred to an excessive extent onto the pantie interior following its separation from a completely silicone-coated surface. Using such a sanitary towel could hence result in considerable damage, in the sense that many women are not prepared to wear panties which are even minimally stained, the word "considerable" being used here in consideration of the fact that the panties concerned are generally luxury articles.

US-A-5972472 relates to a separator having a matte or embossed surface which, according to example 2, concerns a 20 micron thick film constituted by by a polymer blend composed of 50% by weight polyethylene, 45% by weight polypropylene and 5% by weight an hydratation product. The film surface is brought into contact with en embossing roll having a roughness of 10 micron in order to form an embossed surface having a roughness of 10 micron on the film surface.

US-A-6186993 discloses an a wrapping construction of an absorbent article which, according to claim 1 thereof, comprises an absorbing article (2) including a body (3), a pair of wings (4) disposed on opposed left and right lateral sides of said body (3), said body being provided with an adhesive portion (37) on a side of a surface thereof not contacting skin, said wings (4) being provided with an adhesive portion (41) on said side of said body not contacting skin.

GB-A-2273279 relates to an envelope for sanitary towels (10) in which, as described in claims 1 and 2, the towel is folded back in those areas of its surface which do not contact skin, and it is wrapped with a wrapping material (20), whereby the areas of said surface which do not contact skin are provided with an adhesive material (16), a peelable layer (21) being provided in correspondence of the inner area of the surface of the wrapping material.

According to this disclosure the adhesive material is applied by an embossing technique, thereby obaining areas or zones which may well show a different shape, but which imply the important drawback of altering the planarity of the surface the adhesive material is applied upon.

An object of the present invention is to define a envelope for containing sanitary towels (of the type which automatically offers the sanitary towel ready for its application to the pantie interior as a result of being extracted from the containing envelope), which comprises non-stick means attached to the sanitary towel adhesive with a force which can be easily adapted to the specific production requirements and/or to the required adhesiveness of the sanitary towel against the pantie interior. A further object is to define non-stick means, as aforestated, which can implement the interior of the usual polyethylene film, intended to receive the sanitary towel, by economical production methods. A further object is to define non-stick means, as aforestated, which can be applied by the known methods of other sectors, in order to avoid the expense of specific plants. These and further objects will be seen to have been attained on reading the ensuing detailed description, illustrating a process for manufacturing a film suitable for making an envelope for containing adhesive sanitary towels for women having the features described in claim 1, as well as an envelope for containing adhesive sanitary towels for women having the features described in claim 4. The dependent claims outline preferred forms of embodiment of the present invention.

The invention is illustrated by way of non-limiting example in the accompanying drawing in which:
- Figure 1 shows one example of a envelope and its contained sanitary towel in an open configuration showing that side thereof treated with the said dotting;
- Figures 2, 3 4 and 5 show, with an enlargement of about ten times the actual dimensions, how four inner envelope surfaces could appear when "printed" with said silicone fluids.

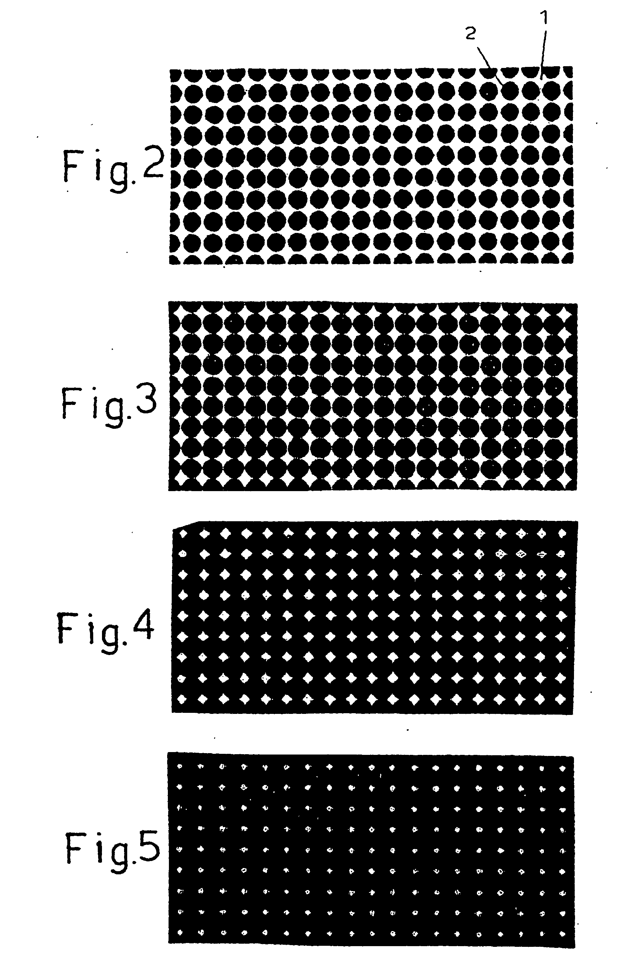

This particular printing is carried out in the form of patterns of uniformly distributed dots, they differing by the different separation between "voids" 1 and "fillings" 2. Assuming that the black circular dots are the fillings 2, indicating silicone applied by usual printing methods, and that the white area external to them indicates the voids 1, i.e. polyethylene film alone, it will be apparent that for equal distances between the centres of these dots, the larger the dots the more they saturate the surface on which they are applied. This is apparent from a comparison of Figures 2, 3, 4 and 5. Other characteristics being equal, it is sufficient to vary the printing reticulation to vary the percentage of the polyethylene surface covered by the silicone or other generic non-stick material. The said method of applying the silicone (or other generic non-stick material) by printing advantageously enables a discontinuity to be created, resulting in extremely uniform silicone distribution.

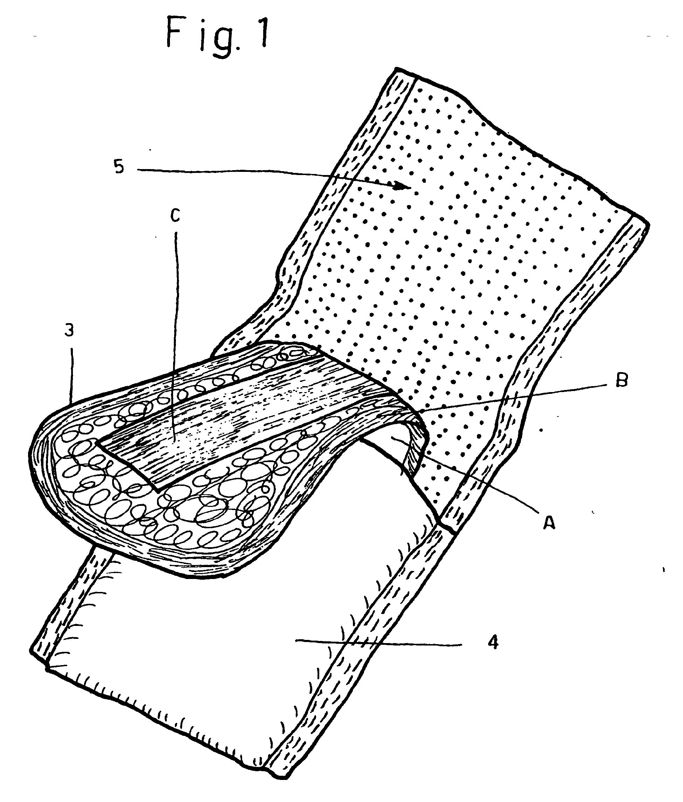

This precise metering also results in considerable saving in silicone; with reference for example to Figure 2 it can be seen that the "white" surface represents about 50% of the total. This would not have been possible with the current known method of applying the silicone by "spreading", which conceptually results in 100% cover. Although in Figures 2, 3, 4 and 5 the "printed" silicone is shown as uniformly distributed circles, silicone application by printing enables other geometrical figures to be used, and their distribution and/or size to be varied. An example of a printed figure different from a circle could be a rhombus; such a figure would in fact be advantageous in differentiating the silicone between the longitudinal axis and transverse axis of the film in reels, used for forming the containing envelopes. Rhomboidal geometry can also be used for differentiating, in the most suitable manner, the non-stick capacity of its inner surfaces relative to the direction of separation with which the sanitary towel is extracted from the envelope (Figure 1). Another advantage of applying silicone by usual printing methods, in particular flexographic and rotogravure printing, is that of being able to offer polyethylene surfaces which are as bare as necessary in order not to hinder the construction of the containing envelope by the usual joining of their edges. Another advantage is the achieving of a dotted distribution of the non-stick material along longitudinal bands of different concentration, based on which region of adhesive present on the sanitary towel has to be reduced to a minimum quantity, or to a minimum adherence value, in order not to cause adhesive residues to be released onto the pantie following the final removal of the sanitary towel.

The fluid silicone applied by said printing methods can be of various types; an example of characteristics suitable for its application to the invention is offered by the product TEGO RC 711 produced by GOLDSCHMIDT or the product UV9300 GE BAYER associated with the UV9380 C GE BAYER catalyst. Although the aforesaid silicone is one of the non-stick materials most used in protecting or isolating the adhesive surfaces of the adhesives generally used in the field of the invention, this does not imply that the invention refers exclusively to these. In this respect, surface silicone-coating is to be understood principally as a non-stick treatment. Consequently, in this sense, different printing liquids which achieve analogous non-stick properties must also be considered as falling within the scope of the invention, for example liquids with characteristics similar to printing inks suitable for fixing PTFE powder onto the polyethylene surface. In this respect, what is important is the use of a method by which non-stick products are applied with dotted distribution, determined by printing reticulation methods, to that polyethylene surface which is to form the interior of the containing envelope for sanitary towels provided with adhesive.

## Claims

1. Process for manufacturing a film suitable for making an envelope for containing adhesive sanitary towels for women **characterised in that** said film is wound in reels and **in that** the side of said film which is to form the envelope inner surface (5) is printed, preferably by usual flexographic or rotogravure methods, with liquids having physical and chemical properties which render them similar to the usual printing inks, said liquids being able to stably deposit, on the treated film, suitable quantities of non-stick materials, preferably of silicone nature, in a dotted form which can be freely modified in shape and density.

2. Process as claimed in claim 1, **characterised in that** the dotted configuration of non-stick material, advantageously silicone, consists of a plurality of small circles the diameter of which can be varied to vary, for equal distances between their axes, the free space not covered by them on the basis of the degree of adhesion required of the resultant surface.

3. Process as claimed in claim 1, **characterised in that** the dotted configuration of non-stick material, indicatively silicone, consists of a plurality of small rhombi of orderly orientation and distribution, the size of which can be varied to vary, for equal distances between their axes, the free space not covered by them on the basis of the degree of adhesion required of the resultant surface.

4. An envelope, indicatively of a polyethylene film, for containing adhesive sanitary towels for women, whereby the side of said film which is to form the envelope inner surface (5) is provided with a quantity of non-stick material, advantageously of silicone nature, distributed in dotted manner, so that those surface regions which do not contain it offer a removal and retention action on excess adhesive and generally a reduction in the adherence of the usual adhesives applied to the sanitary towel, **characterised in that** the non-stick material is spread on said side of said film by means of usual printing methods, in particular flexographic and rotogravure printing.

5. An envelope according to claim 4, **characterised in that** the dotted distribution of the non-stick material is of varying density.

6. An envelope according to claim 5, **characterised in that** the dotted distribution of the non-stick material is to form bands of different gradations in order to offer, when subsequently positioned in the inner part (5) of the envelope, a differential grip on the adhesive present on one side of the sanitary towel, for the purpose of facilitating the retention of excess adhesive in those regions which could most damage the panties should it become transferred to them.

## Patentansprüche

1. Verfahren zur Produktion einer zum Herstellen einer Hülle zum Aufnehmen selbstklebender Damenbinden geeigneten Folie, **dadurch gekennzeichnet, dass** die Folie aufgewickelt wird, und dass die die Innenfläche (5) der Hülle bildende Seite der Folie, vorzugsweise mit Flexodruck- oder Tiefdruckverfahren, mit Flüssigkeiten bedruckt wird, welche physikalische und chemische Eigenschaften haben, welche sie den gewöhnlichen Druckfarben ähnlich machen, wobei die Flüssigkeiten geeignet sind, auf die bearbeitete Folie geeignete Mengen von, vorzugsweise silikonartigen, Antihaft-Materialien in einer gepunkteten in Gestalt und Dichte frei veränderbaren Form stabil aufzubringen.

2. Verfahren nach Anspruch 1, **dadurch gekennzeichnet, dass** die punktweise Anordnung des Antihaft-Materials, vorzugsweise Silikon, aus einer Vielzahl kleiner Kreise besteht, welche einen veränderbaren Durchmesser aufweisen, um bei gleichen Abständen zwischen deren Achsen den nicht von diesen bedeckten Freiraum auf der Grundlage des Grads der für die resultierende Fläche erforderlichen Haftung zu verändern.

3. Verfahren nach Anspruch 1, **dadurch gekennzeichnet, dass** die punktweise Anordnung des Antihaft-Materials, insbesondere Silikon, aus einer Vielzahl geordnet ausgerichteter und verteilter kleiner Rhomben besteht, welche eine veränderbare Größe aufweisen, um bei gleichen Abständen zwischen deren Achsen den nicht von diesen bedeckten Freiraum auf der Grundlage des Grads der für die resultierende Fläche erforderlichen Haftung zu verändern.

4. Hülle, insbesondere aus einer Polyäthylen-Folie, zum Aufnehmen selbstklebender Damenbinden, wobei die die Innenfläche (5) der Hülle bildende Seite der Folie mit einer Menge eines, vorzugsweise silikonartigen, Antihaft-Materials versehen ist, welches punktartig verteilt ist, so dass die Flächenbereiche, welche dieses nicht enthalten, ein Entfernen und ein Zurückhalten eines Überschusses an Klebstoff und allgemein eine Reduktion der Haftung gewöhnlicher bei der Binde verwendeter Klebstoffe ermöglichen, **dadurch gekennzeichnet, dass** das Antihaft-Material auf die Seite der Folie mit herkömmlichen Druckverfahren, insbesondere Flexodruck oder Tiefdruck, aufgebracht ist.

5. Hülle nach Anspruch 4, **dadurch gekennzeichnet, dass** die Dichte der punktweisen Verteilung des Antihaft-Materials variiert.

6. Hülle nach Anspruch 5, **dadurch gekennzeichnet, dass** die punktweise Verteilung des Antihaft-Materials unterschiedlich abgestufte Bereiche ausbildet, um beim nachfolgenden Anordnen im Innenteil (5) der Hülle eine unterschiedliche Griffigkeit für den auf einer Seite der Binde vorhandenen Klebstoff zu ermöglichen, um das Zurückhalten von überschüssigem Klebstoff in den Bereichen zu erleichtern, welche den Slip am stärksten beschädigen könnten, wenn er auf ihn übertragen wird.

## Revendications

1. Procédé de fabrication d'un film convenant à la fabrication d'une enveloppe destinée à contenir des serviettes hygiéniques auto-adhésives pour dames, **caractérisé en ce que** ledit film est enroulé en bobines et **en ce que** le côté dudit film devant constituer la surface interne de l'enveloppe (5) est imprimé, de préférence selon des procédés courants de flexographie ou de rotogravure, avec des liquides ayant des propriétés physiques et chimiques les rendant similaires aux encres d'impression habituelles, lesdits liquides étant capables de déposer de manière stable, sur le film traité, des quantités adéquates de matériaux non collants, de préférence de type silicone, dans une forme en pointillé, dont on peut librement modifier la forme et la densité.

2. Procédé selon la revendication 1, **caractérisé en ce que** la configuration en pointillé du matériau non collant, avantageusement du silicone, se compose d'une pluralité de petits cercles dont le diamètre peut varier pour modifier, pour des distances égales entre leurs axes, l'espace libre non recouvert par ceux-ci sur la base du degré d'adhérence requis de la surface résultante.

3. Procédé selon la revendication 1, **caractérisé en ce que** la configuration en pointillé de matériau non collant, par exemple en silicone, consiste en une pluralité de petits losanges d'orientation et de répartition ordonnées, dont la taille peut varier pour modifier, pour des distances égales entre leurs axes, l'espace libre non recouvert par ceux-ci sur la base du degré d'adhérence requis de la surface résultante.

4. Enveloppe, par exemple en film de polyéthylène, destiné à contenir des serviettes hygiéniques auto-adhésives pour dames, où le côté dudit film devant former la surface interne d'enveloppe (5) est muni d'une quantité de matériau non collant, avantageusement de type silicone, réparti en pointillé, de sorte que les régions superficielles ne le contenant pas réalisent une action de dépose et de rétention sur un adhésif en surplus et généralement une baisse de l'adhérence des adhésifs habituels appliqués à la serviette hygiénique, **caractérisée en ce que** le matériau non collant est étalé sur ledit côté dudit film à l'aide de procédés d'impression habituels, en particulier une impression par flexographie et par rotogravure.

5. Enveloppe selon la revendication 4, **caractérisée en ce que** la répartition en pointillé du matériau non collant est de densité variable.

6. Enveloppe selon la revendication 5, **caractérisée en ce que** la répartition en pointillé du matériau non collant doit former des bandes de différentes gradations pour constituer, après positionnement dans la partie interne (5) de l'enveloppe, une prise différentielle sur l'adhésif présent sur un côté de la serviette hygiénique, pour faciliter la rétention d'un surplus d'adhésif dans les régions risquant le plus d'endommager la culotte en cas de transfert sur celle-ci.
